# EUROPEAN PATENT SPECIFICATION

(11) **EP 2 611 454 B1**
(45) Date of publication and mention of the grant of the patent: **25.03.2015**
(21) Application number: 10763851.2
(22) Date of filing: 02.09.2010
(51) Int. Cl.: A61K 36/48, A61P 1/00

(54) **PERORALLY APPLICABLE PREPARATION CONTAINING HISTAMINASE OF VEGETABLE ORIGIN, RESISTANT TO PEPSIN AND TRYPSIN AND A PROCESS FOR PRODUCING IT**
PERORAL ANWENDBARE ZUBEREITUNG MIT EINER PEPSIN- UND TRYPSINRESISTENTEN HISTAMINASE PFLANZLICHEN URSPRUNGS UND HERSTELLUNGSVERFAHREN DAFÜR
PRÉPARATION APPLICABLE PAR VOIE PERORALE CONTENANT DE L'HISTAMINASE D'ORIGINE VÉGÉTALE, RÉSISTANTE À LA PEPSINE ET LA TRYPSINE ET UN PROCÉDÉ POUR PRODUIRE CELLE-CI

(43) Date of publication of application: 10.07.2013
(73) Proprietor: Debreceni Egyetem, 4032 Debrecen (HU)
(72) Inventor: SIPKA, Sándor, H-4032 Debrecen (HU); GYÖRY, Zoltán, H-4032 Debrecen (HU); BORBÉLY, Jánosné, H-4032 Debrecen (HU); KERESZTES, Tamás, H-4025 Debrecen (HU); VÁRNAI, Péter, H-1125 Budapest (HU); BÍRÓ, Tamás, H-4028 Debrecen (HU)
(74) Representative: Varnai, Aniko
(86) International application number: PCT/HU2010/000094
(87) International publication number: WO 2012/028891

(56) References cited:
- WO-A2-02/43745
- DATABASE WPI Week 201104 Thomson Scientific, London, GB; AN 2011-A23958 XP002631128, & HU 0 900 157 A1 (DEBRECENI EGYETEM) 28 December 2010 (2010-12-28) & HU 0 900 157 A2 (DEBRECENI EGYETEM [HU]) 28 December 2010 (2010-12-28)
- CUZZOCREA SALVATORE ET AL: "Plant histaminase as an investigational drug in splanchnic artery occlusion and reperfusion.", August 2008 (2008-08), EXPERT OPINION ON INVESTIGATIONAL DRUGS AUG 2008 LNKD- PUBMED:18616412, VOL. 17, NR. 8, PAGE(S) 1151 - 1160, XP002628207, ISSN: 1744-7658 page 1153
- MCGUIRL M A ET AL: "Purification and characterization of pea seedling amine oxidase for crystallization studies.", November 1994 (1994-11), PLANT PHYSIOLOGY NOV 1994 LNKD- PUBMED:7824646, VOL. 106, NR. 3, PAGE(S) 1205 - 1211, XP002628208, ISSN: 0032-0889 the whole document
- SURESH M R ET AL: "DIAMINE OXIDASE OF LATHYRUS SATIVUS SEEDLINGS PURIFICATION AND PROPERTIES", JOURNAL OF BIOSCIENCES, INDIAN ACADEMY OF SCIENCES, IN, vol. 1, no. 2, 1 June 1979 (1979-06-01), pages 109-124, XP001066515, ISSN: 0250-5991, DOI: DOI:10.1007/BF02706323
- MASINI EMANUELA ET AL: "Pea seedling histaminase as a novel therapeutic approach to anaphylactic and inflammatory disorders. A plant histaminase in allergic asthma and ischemic shock", THE SCIENTIFIC WORLD JOURNAL, THE SCIENTIFICWORLD LTD, GB, NL, vol. 7, 1 January 2007 (2007-01-01), pages 888-902, XP009145942, ISSN: 1537-744X

## Description

The invention relates to a perorally applicable preparation containing histaminase of vegetable origin resistant to pepsin and trypsin and a process for producing it.

The preparation according to the invention can be dosed perorally to improve digestion, to reduce appetite, as well as for the purpose of weight reduction. The preparation is structurally resistant to trypsin, acting in the duodenum, and it is also protected against pepsin, the protein decomposing enzyme of stomach. The preparation can be applied with a high efficiency, as the enzyme remains active for a long time in the intestine to decompose histamine, causing unpleasant side effects connected with eating.

Under natural circumstances histamine is mainly decomposed by histaminase in the duodenum produced by the pancrease whereas also pepsin is entering the duodenum from the stomach.

Pepsin entered the duodenum can decompose histaminase at a certain extent and reduces its activity.

This fact causes a great problem for many people suffering from an "intolerance to histamine" or "hyperacidity" caused by the high level of histamine.

The "intolerance to histamine" is a pathological status arising on the base of the increased histamine quantity in the digestive system and/or the reduced ability to decompose it, what is frequently accompanied with hyperacidity or abundant gastric acid production.

Histamine is an important type of biogenic amines with a great number of strong biological effects.

It is, among others, one of the most active agents increasing the production of gastric juice and the feeling of hunger improving appetite.

In healthy persons, histamine is quickly decomposed by "histaminase" called biochemically diamine oxidase enzyme (DAO) being also a natural component of intestinal juice.

The natural decomposition of histamine takes place in the small intestine according to the following chemical reactions:

a.) RCH₂NH₂ (histamine) + O₂ + H₂O + histaminase → RCHO + H₂O₂ + NH₃

b.) 2H₂O₂ + catalase → 2H₂O + O₂

When activity of DAO is low or its quantity is small, a so-called "histamine intoxication" can occur accompanied with serious and unpleasant symptoms. Histamine is continuously produced in different organs, and it is the mediator of various unpleasant symptoms in the organism.

There are food products rich of histamine (e.g. cheese sorts, fish sorts, sausage products etc.) and alcoholic drinks (particularly some sorts of red wine) or drugs inhibiting activity of DAO (antiphlogistic drugs, painkillers etc.) or food products provoking the release of histamine (lemon juice, tomato, chocolate, some fish foods, liqueurs, spices etc.) causing diarrhea, headache, conjunctivitis, rhinitis, reduction of blood pressure, disturbances of hearth rhythm, erythema and itching accompanied with "heartburn" (Maintz L. and Novak N. Histamine and histamine intolerance. Amer J Clin Nutr 2007; 5. p.1185-1196).

There is about 1% of the population suffering from histamine intolerance, 80% of them is in the medium age. This proportion can affect approximately 100 000 persons in Hungary, 5 million persons in Europe and 60 million persons in the whole world.

In order to the reduce unpleasant symptoms caused by histamine intolerance, there is the possibility to introduce histaminase enzyme (DAO) perorally into the intestinal tract mainly to decompose histamine either entering the small intestine together with the food or being induced by the food.

Earlier it was already tried to apply capsules containing histaminase enzyme of animal origin extracted from pig kidney.

This preparation contained histaminase enzyme of animal origin and stabilizing agents. Nevertheless, the preparation has not got widespread application (Lorenz W, Ennis M, Doenicke A, Dick W.: Perioperative uses of histamine antagonists. J Clin Anesth 1990; 2: 345-60).

Similarly to the other DAO-s of mammalian origin, this histaminase enzyme of animal origin is also a dimerized molecule consisting of two chains of 90 kilodalton each, and it has the total molecular weight of 180 kD, it is resistant to trypsin but sensitive to pepsin.

Pepsin splits it into pieces with a high activity.

It was observed that pepsin splits the DAO enzymes of animal origin at a high extent (Houen G, Jorgensen J, Leonardsen L, Larsson LI.: Purification and partial characterization of mammalian Cu-dependent amine oxidizes. Acta Chem Scand 1993:47:902-909; Kleutz MD, Adamsons K, Flynn JE Jr. Optimized preparation and determination of pea seeding amine oxidase. Prep Biochem 10: 615-631; McGuirl MA, McCahon CD, McKeown KA, Dooley DM.: Purification and characterization of pea seedling amine oxidase for crystallization studies. Plant Physiol 1994; 106: 1205-1211).

The patent description EP 1339422 describes an application of histaminase of vegetable origin purified by fractionation for the treatment of allergic and septic shocks.

Histaminase is produced from pea plant (Pisum sativum L.) or from plants of Lens Culinaris, Cicer arietinum, Latirus sativus by extraction then by the purification of extracts.

The preparation is administered in the forms of injection or spray administered into blood, abdomen, trachea or bronchi.

During our experiments, however, we experienced that the preparation cannot be applied perorally, because pepsin decomposes it either in the stomach or in the duodenum, so it becomes ineffective or its effect is very small.

We also found during the experiments that the frequent application of the preparation into blood or airways can cause unpleasant immune reactions.

We recognized that the pressed vegetable juice obtained from the sprouts of pea (Pisum sativum L.), lentil (Lens culinaris); chick pea (Cicer arietinum); grass pea (Latirus sativus); bean (Phaseolus vulgaris) or field bean (Vicia fava) or from a mixture of sprouts of the mentioned plants by pressing contains not only histaminase (DAO) of vegetable origin, resistant to trypsin, but it contains also catalase and a protease (pepsin) inhibitor substance, as well.

The preparation according to the invention keeps almost its histamine decomposing ability totally even in presence of pepsin, and it keeps steadily this effect in the whole alimentary tract, as well.

The subject of this invention is a perorally applicable preparation of vegetable origin, resistant to pepsin and trypsin, based on histaminase, optionally for the use as an agent for improving digestion, reducing appetite or reducing body weight The characteristics of the preparation according to the invention are as follows, it is a pressed juice - which is optionally in lyophilized form - obtained from seedlings of pea (Pisum sativum L.), lentil (Lens culinaris), chick pea (Cicer arietinum), grass pea (Latirus sativus), bean (Phaseolus vulgaris) or field bean (Vicia fava) or - from a mixture of seedlings of the above mentioned plants which contains histaminase, catalase and protease inhibitor.

The preparation contains 0.1 to 2.5 mass% of histaminase, 0.1 to 2.5 mass% of catalase and 0.5 to 3.2 mass% of protease inhibitor calculated to dry substance. The preparation according to the invention can also be applied as a digestion improving product, an appetite reducing product, as well as a weight reducing product.

The subject as well of the invention is the production of the above preparation.

The preparation of the invention is produced in such a way that the seeds of pea (Pisum sativum L.), lentil (Lens culinaris); chick pea (Cicer arietinum), grass pea (Latirus sativus), bean (Phaseolus vulgaris) or field bean (Vicia fava) or a mixture of the seeds of the above mentioned plants are selected, disinfected, soaked in water, then let them sprout, then the seedlings are separated, the sprouts are refrigerated to a temperature between -10°C and -20°C, let to stay at this temperature, then let to warm up to 4 to 10°C, the material is pressed, the pressed juice is separated and optionally lyophilized.

It is advantageous to take it daily at least in the dose of 0.2 mg/kg body weight as a food complementing preparation (calculated to dry substance).

During our experiments, we determined the molecular mass of histaminase being present in the pressed juice prepared from the sprouts of the above mentioned plants.

It is approximately 2 x 75 = 150 kD indicating that this molecule of vegetable origin has a structure different from the DAO-s of mammalian origin having the molecular mass of 2 x 90 = 180 kD.

The molecular mass of catalase being present in the pressed juice is 4 x 57 = 228 kD.

The pressed juice according to the invention, as well as its lyophilized product are preparations, able for peroral administration in the following forms: a) as a enterosolvent capsules (solved in the small intestine), b) as a powder (tablet) applied directly into the stomach because of its resistance to pepsin. Thus, it can be used more advantageously and economically than all the known preparations of animal or vegetable origins to decompose histamine and to reduce histamine intolerance accompanied often with hyper-acidity, furthermore, to reduce the increased appetite and overweight.

We have marked the preparation according to the invention as "PTRNH".

The process of histamine decomposition takes place in presence of PTRNH either in the stomach or in the duodenum in the following way:

a.) RCH₂NH₂ (histamine) + O₂ + H₂O + PTRNH (DAO component) → RCHO + H₂O₂ + NH₃

b.) 2H₂O₂ + PTRNH (catalase component) → 2H₂O + O₂

As the main process of digestion of the histamine containing foods is taking place in the small intestine, and the release of histamine from them also occurs here, although a smaller part of histamine is already released in the stomach, the reduction of histamine level can be achieved by the oral administration of the capsules or tablets solved in the stomach or in the small intestine, or by the pressed juice containing PTRNH according to invention administered immediately perorally.

The preparation according to the invention resists not only to trypsin being present in the small intestine but also to pepsin being present in the stomach or entering the small intestine, as well.

We recognized that our preparation being administered perorally is more efficient than any other perorally administered histaminase preparations of either animal or vegetable origins to decompose histamine in the digestive system produced by different reasons and having unpleasant side effects even by that reason that it contains also catalase and protease inhibitor (pepsin inhibitor) components in parallel to histaminase resistant to trypsin because of its original nature.

It is known that histamine produced in the small intestine is also a natural stimulus for the production of gastric acid.

The reduction its quantity, results in a reduced production of gastric acid and a diminished feeling of hunger, what existing for a longer period, can inhibit the gain of body weight or it can result in a loss of body weight.

We introduce the preparation according to the invention and its production below.

### Example 1

### Production of PTRNH

### A.) The germination

The seeds of any varieties of pea (Pisum sativum) either for spring-sowing or those for winter sowing are primarily applicable for germination, but seeds of other plants such as lentil, chick pea, grass pea, bean or field bean can also be used, if they fulfill with the following requirements: the germinative ability should be at least 95%, the purity should be at least 98% and it should be grown on an area (arable land) where no synthetic plant protection chemicals were used at least during the last three years, so these areas contain the residues of those chemicals or their decomposed toxic products in the soil layer of 0.001 to 200 cm at a concentration lower than the limit prescribed by the Law.

The seeds to be germinated are grown in such a system of quality control (ISO 22000, HACCP, GAP) wherein the activities can be followed up using the records and documents related to the technology of growing and the operations following the harvest.

### The course of the germination:

The seeds are selected and the damaged grains are removed from the mass, because they either do not germinate or do not form healthy sprout.

The carefully disinfected seeds are then soaked during 8 to 12 hours in distilled water.

The seeds are taking up water in 40 to 45% of their own mass. The seeds "saturated with water" are layered on germinating trays in a thin layer, in the thickness of one seed possibly, then the seeds are covered by wet filter-paper. The trays are put into a germinating thermostat and kept in darkness at room temperature for 2 days. The seeds are watered by distilled water every day in order to prevent drying. The germinating seeds are implanted forming a thick layer into a layer of sand in the depth of 1-10 cm. The sand is disinfected either by washing with acid or by heating at 400°C in a heating furnace, then the germination is carried out in a thermostat at 25°C. The culture is controlled each day at the time of water supplying. It is important to mention that both the external and internal temperatures (in the room) are controlled at constant levels. The germs produced without light do not contain chlorophyll, and their height reaches 20-25 cm within the 10-14 days. The harvesting takes place then, wherein the stems are removed from the seeds and the roots. It is necessary to keep both the hands and the tools disinfected during all procedures.

### B.) The processing of the seedlings

### • Obtaining the material

2.00-2.50 kg of seedlings (the parts above roots), having about 10% of dry material content, are obtained from germinating of 5 kg of pea seeds on the 10^{th} day, depending on the species of the plant. The sprouts are cut.

The cut plant parts are slowly frozen to -20°C in order to facilitate the growing of ice crystals to destroy the cell walls making easier to obtain the intracellular juice of plant in this way. The temperature of -20°C is kept for two hours, then the mass is let to warm up to 4°C. About 70 to 80 mass% of the whole juice can be obtain using a screw-press generally used to produce fruit juices. No filtration is needed thereafter. The optimal working parameter of the screw-press is 10 to 30 N/cm³ for seedlings.

The whole amount of liquid is pressed out from the mass of cut seedlings of high liquid content, then all the pressed liquid is collected in a container. This solution of about 3.5 to 4.0 liter contains histaminase, catalase and the protease inhibitor (pepsin inhibitor) molecules.

The lyophilized form of this liquid is PTRNH. This is already one component of the enterosolvent capsules solved only in the small intestine or of the tablet taken in a raw form into the stomach.

The ratios of pressed juice/dry materials are 70 to 80/20 to 30%.

The quantitative distributions of the main protein components of PTRNH, the histaminase preparation of pepsin and trypsin resistance are as follows in the lyophilized dry substances derived from the raw material of pea (Pisum sativum), lentil (Lens culinaris), chick pea (Cicer arietinum), grass pea (Latirus sativus), bean (Phaseolus vulgaris) and field bean (Vicia fava):

| | |
|---|---|
| Histaminase (diamino oxidase) | 0.1 to 2.5 mass% |
| Catalase: | 0.1 to 2.5 mass% |
| Protease inhibitor (pepsin inhibitor) | 0.2 to 3.2 mass% |

### C.) There are several methods which can be used to measure the enzyme activities of the samples:

a.) Measurement of histamine concentration by fluorometry (for histaminase, DAO),
b.) Measurement of histamine concentration by method ELISA (for histaminase, DAO),
c.) Measurement of histamine analogue "benzil amine" concentration by spectrophotometry (for histaminase, DAO)
d). Measurement of catalase activity on the base of decomposition of H₂O₂ by spectrophotometry.

The proof of the known resistance of histaminase (DAO) to trypsin was based on the measurement of the decomposition of histamine using different concentrations of trypsin.

The pressed juice doses produced according to the example are lyophilized in a known way and the lyophilized products are stored safely at +4°C.

The preparation produced in the mentioned way is marked with the sign of PTRNH.

### Example 2

All is done as in the Example 1 but pure histaminase is produced for experimental purposes from the lyophilized product by fractional precipitation and gel filtration according to the method described in the article of McGuirl MA, McCahon CD, McKeown KA, Dooley DM: Purification and characterization of pea seedling amine oxidase for crystallization studies, Plant Physiol, 106:1205-1211, 1994.

The preparation is marked as SDAO.

### Example 3

All is done as in the Example 1 but a mixture of histaminase and catalase is prepared from the pressed juice by gel filtration of type Sephadex G 100 for experimental purposes.

The preparation is marked as SDAOK.

### Example 4

The proving of resistance of PTRNH against pepsin:

The used components:
Histaminase of pig kidney (DAO, manufacturer: Sigma Aldrich Ltd., HU)
PTRNH Pepsin (manufacturer: Sigma Aldrich Ltd., HU)
Phosphate buffer of pH 3.5, of 0.1 M
Phosphate buffer of pH 7.8, of 0.1 M
Reaction I: in buffer of pH 3.5, 10 minutes at 37°C (measurement of pepsin activity)
   a) Pig DAO (0.1 mg/0.1 ml) + pepsin (0.1 mg/0.1 ml) + 0.2 ml of buffer (the final volume is 0.4 ml)
   b) Pig DAO (0.1 mg/0.1 ml) + 0.3 ml of buffer (the final volume is 0.4 ml)
   c) PTRNH (1 mg/0.1 ml) + pepsin (1 mg/0.1 ml) + 0.2 ml of buffer (the final volume is 0.4 ml)
      (where the histaminase content of PTRNH is 0.1 mg/0.1 ml)
   d) PTRNH (1 mg/0.1 ml) + 0.3 ml of buffer (the final volume is 0.4 ml)
      (where the histaminase content of PTRNH is 0.1 mg/0.1 ml)
      Note: the proportions DAO/pepsin and PTRNH/pepsin proteins were the same in both systems.
Reaction II: in final medium of pH 7.2, the time of treatment is 60 minutes, at 37°C (measurement of histaminase activity)
   Histamine analogue benzamine substrate was added to the samples a; b; c; and d; in the final concentration of 3 mM in 3 ml of phosphate buffer (pH 7.8).
   (The buffer with pH 7.8 changes the reaction medium I. from pH 3.5 on pH 7.2 required for the activity of histaminase.)

The results of the experiments are as follows:

**Table 1**

| | |
|---|---|
| | The proof of the resistance of PTRNH to pepsin compared to a histaminase derived from pig kidney (the effect of pepsin for 10 minutes at 37°C). |

| Samples | Activity of histaminase (%) |
|---|---|
| Histaminase of pig kidney | 100 |
| Histaminase of pig kidney + pepsin | 56 |
| PTRNH | 100 |
| PTRNH + pepsin | 98.5 |

| | |
|---|---|
| 1. The pre-treatment with pepsin caused inhibition of 44% in the DAO of pig kidney. 2. The pre-treatment with pepsin caused inhibition of 1.1% in the PTRNH | |

### Conclusion:

The juice pressed out from the seedling contains histaminase in a pepsin resistant form.

This observation adds new practical advantages to the former knowledge relating to the histaminase of vegetable origin.

This result can be attributed to the fact that the preparation according to the invention also contains catalase decomposing H₂O₂ and a protease inhibitor, in addition to the trypsin resistant histaminase of vegetable origin.

The novelty of the preparations according to this invention is the recognition that there is a component in the not fractionated pressed juice of the seedlings of leguminous plants with histaminase activity, which can inhibit pepsin, thus it is able to protect histaminase from pepsin in the stomach or in the intestinal tract. Thus, the histaminase decomposing effect of the preparation remains active in the intestines for a longer time, eliminating the negative effect of histamine.

This unforeseen effect makes possible that these preparations according to the invention can be effectively applied also orally.

The above mentioned example presents some experimental and numerical results of PTRNH prepared from pea (Pisum sativum L.). Nevertheless, we obtained similar results using preparations made from lentil (Lens culinaris), grass pea (Latirus sativus), bean (Phaseolus vulgaris) or field bean (Vicia fava).

The potent histaminase inhibiting effect of PTRNH preparations introduced into a human intestinal tract can be expected to be about 3 x 3 mg/kg/day.

### Example 5

The effects of various preparations with histaminase content on the reduction of intestine contractions caused by histamine in rats:

### The samples:

I "histaminase" (DAO) obtained from pig kidney (manufacturer: Sigma-Aldrich Ltd., HU)
II Extract of pea containing "histaminase" produced from pressed juice (SDAO) (produced according to the Example 2, analogous to the product described in the patent description EP 1.339.411)
III Extract of pea having "histaminase" and "catalase" content made from pressed juice and purified (SDAOK, according to the Example 3).
IV Preparation made from pressed juice not purified having "histaminase + catalase + protease inhibitor" content (PTRNH according to the Example 1)

### The protocol of examination:

The measurements of the contractions of suspended rat ileum sections were carried out in aerated Tyrode feeding solution (GIBCO, USA) in a water bath chamber of 10 ml, at 37°C testing the effects of histamine solutions pre-treated with various histaminase preparations.

### The pre-treatment of histamine:

a.) 0.1 µg of histamine (Sigma Ltd. Budapest) in 1 ml of Tyrode solution (Pre-incubationor 30 minutes in water bath at 37°C),
b.) 0.1 µg of histamine + pig DAO (0.1 mg) in 1 ml of Tyrode solution (Pre-incubation for 30 minutes in water bath at 37°C),
c.) 0.1 µg of histamine + SDAO (0.1 mg) in 1 ml of Tyrode solution (Pre-incubation for 30 minutes in water bath at 37°C),
d.) 0.1 µg of histamine + SDAOK (0.1 mg) in 1 ml of Tyrode solution (Pre-incubation for 30 minutes in water bath at 37°C),
e.) 0.1 µg of histamine + PTRNH (0.1 mg) in 1 ml of Tyrode solution (Pre-incubation for 30 minutes in water bath at 37°C).

Note: Pig DAO, SDAO, SDAOK and PTRNH all have the same histaminase content i. e. 0.1 mg/ml of pure enzyme content in each system.

The histamine solutions of final volume of 1 ml pre-treated with the different histaminase preparations are added to the contents of organ chambers containing intestine pieces suspended in 9 ml of Tyrode feeding solution (the final volume is 10 ml).

The section of ileum contracts under influence of free histamine and the force of contraction is expressed in milli-Newton (mN) on the base of a computer program.

H₂O₂ is released from histamine decomposed by histaminase having an intestinal irritating effect.

H₂O₂ is decomposed by catalase.

The results are as follows:
a.) 11 mN (under effect of pure, not decomposed histamine)
b.) 9 mN (under effect of partially decomposed histamine and released H₂O₂ using DAO of pig kidney),
c.) 7 mN (under effect of partially decomposed histamine and released H₂O₂ using SDAO),
d.) 5 mN (under effect of partially decomposed histamine and decomposed H₂O₂ using SDAOK),
e.) 2 mN (under effect of partially decomposed histamine and decomposed H₂O₂ using PTRNH)

### Conclusions:

The greatest contraction can be measured under effect of not decomposed histamine;

The pre-treatment with purified pig histaminase or pea one (DAO) (i. e. decomposition of histamine) reduces the intestine contracting ability of the preparations;

The presence of catalase together with histaminase continues to reduce the intestinal contracting ability of the solution (in the cases of SDAOK or PTRNH);

The pre-treatment with PTRNH showed the best intestinal protecting effect.

The PTRNH preparations obtained from lentil, chick pea, grass pea bean or field bean showed approximately similar good "intestine protecting" effect against histamine, in addition to that obtained from pea.

### Example 6

The effect of PTRNH and the mixture of "histaminase + catalase" of animal origin to the intestine contraction caused by histamine in presence of pepsin or without it.

### The materials:

a.) A preparation containing "histaminase +catalase + protease inhibitor" of vegetable origin produced from pressed juice without purification (PTRNH according to the Example 1),
b.) histaminase produced from pig kidney (DAO, manufacturer: Sigma-Aldrich Ltd., HU) + catalase made from bovine liver (manufacturer: Sigma-Aldrich Ltd., HU)
c.) pepsin (manufacturer: Sigma-Aldrich Ltd., HU)
d.) histamine (manufacturer: Sigma-Aldrich Ltd., HU)

### The examining system:

Measurement of contractions of rat ileum sections suspended in aerated Tyrode (GIBCO, USA) feeding solution in water bath chamber of 10 ml at 37°C under the influence of histamine solutions pre-treated in various ways, as follows:
a.) 0.1 µg of histamine in 1 ml of Tyrode solution (pre-incubation for 30 minutes in a water bath of 37°C),
b.) 0.1 µg of histamine + PTRNH (10 mg) in 1 ml of Tyrode solution (pre-incubation for 30 minutes in a water bath of 37°C),
c.) 0.1 µg of histamine + PTRNH (10 mg) + pepsin (10 mg) in 1 ml of Tyrode solution (pre-incubation for 30 minutes in a water bath of 37°C),
d.) 0.1 µg of histamine + histaminase from pig kidney (0.1 mg) + bovine catalase (0.1 mg) in 1 ml of Tyrode solution (pre-incubation for 30 minutes in a water bath of 37°C),
e.) 0.1 µg of histamine + histaminase from pig kidney (0.1 mg) + bovine catalase (0.1 mg) + pepsin (0.2 mg) in 1 ml of Tyrode solution (pre-incubation for 30 minutes in a water bath of 37°C).

### Notes:

The protein proportions of histaminase + catalase to pepsin and PTRNH to pepsin were the same in both systems.

The histamine solutions of 1 ml final volume and pre-treated with different preparations are added to the contents of organ chambers containing the intestine pieces suspended in Tyrode feeding solutions of 9 ml (the final volumes were 10 ml).

The ileum intestine section contracts under influence of free histamine and the force of this contraction is expressed in milli-Newtons on the base of a computer program.

H₂O₂ is released from the histamine decomposed by histaminase, having also intestinal irritating effects.

H₂O₂ is decomposed by catalase.

Protease inhibitor inhibits pepsin to decompose histaminase, thus more histamine remains in the solution, with a stronger intestine contracting effect.

### The results are as follows:

a.) 11 mN (under effect of pure, not decomposed histamine),
b.) 3 mN (under effect of partially decomposed histamine and completely decomposed H₂O₂), (PTRNH),
c.) 3.5 mN (under effect of partially decomposed histamine and completely decomposed H₂O₂; pepsin inhibitor has minimum effect on PTRNH),
d.) 8 mN (under effect of histamine decomposed in small extent and decomposed H₂O₂) (DAO of animal origin + catalase),
e.) 10.5 mN (the extent of decomposition of histamine is very small because of the histaminase decomposing effect of pepsin, and catalase decomposes small quantity of H₂O₂) (DAO of animal origin + catalase + pepsin)

The preparations obtained by pressing from the seedlings of lentil, chick pea, bean or field bean show approximately similar good "intestinal protecting" effects against histamine also in the presence of pepsin, as those obtained from pea.

### Example 7

### Examination of appetite reducing effect of the mixture of PTRNH, histaminase and catalase of animal origin in mice

### The samples:

I Watering flacon containing daily exchanged tap water.
II Watering flacon containing daily exchanged tap water and 0.2 mg per 50 ml of mixture of histaminase (from pig kidney) + catalase (from bovine liver) of animal origin purified by the manufacturer,
III Watering flacon containing daily exchanged tap water and 10 mg per 50 ml of PTRNH (pressed histaminase + catalase + enzyme inhibitor of vegetable origin).
IV Watering flacon containing daily exchanged tap water and 0.1 mg per 50 ml of home purified histaminase obtained from pea seedlings (SDAO).

Each mouse drank protein equivalent to 5 to 10 g of pure histaminase in average each day in both systems, which is equivalent to 0.2 to 0.4 □g per kg of pure enzyme quantity in both systems.

### The experimental animals:

I 7 Balb/c male mice aged 8 weeks, which drank tap water (control animals).
II 7 Balb/c male mice aged 8 weeks, which drank tap water containing purified DAO enzyme of animal origin.
III 7 Balb/c male mice aged 8 weeks, which drank tap water containing PTRNH.
IV 7 Balb/c male mice aged 8 weeks, which drank tap water containing SDAO histaminase obtained from pea.

The animals were kept during 30 days in an animal house.

The food consumption was precisely measured and registered each day for each animal.

**Table 2**

| Proof of the appetite reducing effect of PTRNH dosed in the drinking water in a 30 days long animal experiment on Balb/c male mice aged of 8 weeks. | |
|---|---|
| Groups of animals | Consumed food (g per mouse) |
| Mice drinking tap water exchanged every day (n=7) | 156 |
| Mice drinking tap water containing purified DAO + catalase of animal origin exchanged every day (n=7) | 151 |
| Mice drinking 20 □g/ml of PTRNH in tap water exchanged every day (n=7) | 134 |
| Mice drinking tap water containing SDAO extracted from pea exchanged every day (n=7) | 153 |

### The results are as follows:

1. From the second week of the experiment, the animals watered with PTRNH solution consumed less food than the rest of animals.
2. There was no difference between the food consumptions of animals receiving only water, purified mixture of histaminase + catalase of animal origin or purified histaminase.

We can draw the following conclusions:
The preparation made by pressing containing histaminase + catalase + enzyme inhibitor reduces the food intake when it was introduced perorally into the digestive system.
This effect was not available either using purified histaminase extracted from pea (SDAO) or purified mixture of histaminase (DAO) + catalase of animal origin.

The preparations PTRNH obtained by pressing from seedlings of lentil, grass pea, bean or field bean, in addition to that obtained from pea, gave approximately similar appetite reducing effects.

The appetite reducing effect of PTRNH introduced into the intestinal tract can be expected at a dose of 3 x 3 mg/kg/day for humans.

### Example 8

### Examination on the effects of PTRNH and the mixture of histaminase + catalase of animal origin on the inhibition of gaining weight in mice

### The samples:

I Watering flacon containing daily exchanged tap water,
II Watering flacon containing daily exchanged tap water and 0.2 mg per 50 ml of mixture of histaminase (from pig kidney) +catalase (from bovine liver) of animal origin purified by the manufacturer.
III Watering flacon containing daily exchanged tap water and 10 mg per 50 ml of PTRNH (pressed histaminase + catalase + enzyme inhibitor of vegetable origin).
IV Watering flacon containing daily exchanged tap water and 0.1 mg per 50 ml of home purified histaminase obtained from pea seedlings (SDAO).

Each mouse drank protein equivalent to 5-10 µg of pure histaminase in average each day in both systems, which is equivalent to 0.2 to 0.4 µg per kg of pure enzyme quantity in both systems.

### The experimental animals:

I 7 Balb/c male mice aged 8 weeks, which drank tap water (control animals).
II 7 Balb/c male mice aged 8 weeks, which drank tap water containing two purified enzymes of animal origin.
III 7 Balb/c male mice aged 8 weeks, which drank tap water containing PTRNH.
IV 7 Balb/c male mice aged 8 weeks, which drank tap water containing purified histaminase obtained from pea.

The animals were kept for 30 days in an animal house.

The body weight was precisely measured and registered each day for each animal.

**Table 3**

| Proof of the body weight reducing effect of PTRNH dosed in the drinking water in a 30 days long animal experiment on Balb/c male mice aged of 8 weeks. | |
|---|---|
| Groups of animals | Gaining of body weight (g per mouse) |
| Mice drinking tap water exchanged every day (n=7) | 2.2 |
| Mice drinking tap water containing purified DAO + catalase of animal origin exchanged every day (n=7) | 2.3 |
| Mice drinking 20 µg/ml of PTRNH in tap water exchanged every day (n=7) | 1.9 |
| Mice drinking tap water containing SDAO extracted from pea exchanged every day (n=7) | 2.1 |

### The results are as follows:

The gain in weight of animals watered with PTRN solution was less during 30 days than that of animals watered with tap water only or those receiving mixture of purified DAO + catalase of animal origin or purified histaminase (SDAO) obtained from seedlings of pea.

Approximately similar inhibiting effect on gaining weight can be observed dosing PTRNH preparations obtained by pressing from seedlings of lentil, grass pea, bean or field bean.

The inhibiting effect of PTRNH preparations introduced into the intestinal tract on gaining weight can be expected at the dose of 3 x 3 mg/kg/day for humans.

## Claims

1. A perorally applicable preparation of vegetable origin, optionally for the use as an agent for improving digestion, reducing appetite or reducing body weight **characterized in** that, the preparation is a pressed juice - which is optionally in lyophilized form - obtained from seedlings of pea (Pisumsativum L.), lentil (Lens culinaris), chick pea (Cicerarietinum), grass pea (Latirussativus), bean (Phaseolus vulgaris) or field bean (Viciafaba) or from a mixture of seedlings of the above mentioned plants, said preparation contains histaminase that is resistant to pepsin and trypsin, catalase and a protease inhibitor.

2. The preparation according to the Claim 1 **characterized in** that, it contains 0.1 to 2.5 mass% of histaminase, 0.1 to 2.5 mass% of catalase and 0.5 to 3.2 mass% of protease inhibitor calculated to dry material.

3. A process for producing the preparation according to the Claim 1 **characterized in** that, the seed of pea (Pisumsativum L.), lentil (Lens culinaris), chick pea (Cicerarietinum), grass pea (Latirussativus), bean (Phaseolus vulgaris) or field bean (Viciafaba) or a mixture of seeds of the above mentioned plants are selected, disinfected, soaked in water, then let to germinate, the seedlings are separated, the sprouts are frozen to and kept at a temperature between -10 and -20 °C, then let to warm up to 4 to 10 °C, then pressed, and the pressed juice is separated and optionally lyophilized.

## Patentansprüche

1. Ein Präparat der pflanzlichen Ursprung zum Einnehmen durch Mund oder wahlweise zur Anwendung als Wirkstoff für Verbesserung der Verdauung, zur Verminderung der Appetit oder für Verminderung des Körpergewichts, damit **gekennzeichnet dass** das Präparat ein ausgepresster Saft ist oder wahlweise er gefriergetrocknet ist - es aus Samenpflanzen der Erbsen (Pisumsativum L.), Linsen (Lens culinaris), Kichererbsen (Cicerarietinum), Saatplatterbsen (Latirussativus), Bohnen (Phaseolus vulgaris) oder Ackerbohnen (Viciafaba) oder aus eine Mischung der Samenpflanzen der erwähnten Pflanzen gewonnen ist, und das erwähnte Präparat enthält eine gegen Pepsin, Tripsin widerstehende Histaminase, Katalase und einen Inhibitor der Protease.

2. Das Präparat gemäß dem Anspruch 1 damit **gekennzeichnet dass** es zum trockenen Material gerechnete 0.1 bis 2.5 Massen% der Histaminase 0.1 bis 2.5 Massen% der Katalase und 0.5 bis 3.2 Massen% des Inhibitors der Protease enthält.

3. Ein Verfahren für Herstellung des Präparats gemäß dem Anspruch 1 damit gekennzeichnet dass die Samen der der Erbsen (Pisumsativum L.), Linsen (Lens culinaris), Kichererbsen (Cicerarietinum), Saatplatterbsen (Latirussativus), Bohnen (Phaseolus vulgaris) oder Ackerbohnen (Viciafaba) oder die Mischung der Samen der erwähnten Pflanzen ausgewählt, desinfiziert und im Wasser durchnässt werden, denn sie keimen gelassen werden, denn die Samenpflanzen separiert werden, die Sprösse gefroren und in eine Temperatur von -10 bis -20°C gehalten werden, dann sie sich aufwärmen gelassen von 4 bis 10°C, dann gepresst werden, und der ausgepresster Saft separiert und wahlweise gefriergetrocknet wird.

## Revendications

1. Une préparation de origine végétale applicable par voie buccale ou optionnellement comme un agent à améliorer la digestion, à diminuer l'appétite ou à diminuer le poids du corps **caractérisée** par que la préparation est un jus pressé - ou optionnellement dans une forme lyophilisée - obtenue de plantes de semis de pois (Pisumsativum L.) de lentille (Lens culinaris), pois chiche (Ciceraretinum), gesse blanche (Latirussativus), haricot (Phaseolus vulgaris) ou féverole commune (Viciafaba) ou d' un mélange des germes des plantes mentionnés ci-dessus, la préparation mentionnée contient histaminase résistante à pepsine, trypsine, catalase et inhibiteur de protease.

2. La préparation selon la Révendication 1 **caractérisée** par ce qu'elle contient histaminase en 0.1 à 2.5% de masse, catalase en 0.1 à 2.5% de masse, et inhibiteur de protease en 0.5 à 3.2% de masse tout calculé à la matière sèche.

3. Un procédé à préparer la préparation selon la Revendication 1 **caractérisé** par que les graines de pois (Pisumsativum L.), lentille (Lens culinaris), pois chiche (Ciceraretinum), gesse blanche (Latirussativus), haricot (Phaseolus vulgaris) ou féverole commune (Viciafaba) ou le mélange des graines des plantes mentionnées sont triées, désinfectées, macérées dans l'eau, ensuite elles sont laissées à germer, ensuite les plantes de semis sont séparées, les pousses sont gelées et gardées à une température de -10 à -20°C, ensuite elles sont laissées à dégourdir à une température de 4 à 10°C, ensuite elles sont pressées et le jus pressée est séparé et optionnellement lyophilisé.
